# EUROPEAN PATENT SPECIFICATION

(11) **EP 3 299 473 B1**
(45) Date of publication and mention of the grant of the patent: **21.10.2020**
(21) Application number: 16796687.8
(22) Date of filing: 10.05.2016
(51) Int. Cl.: C12Q 1/6883

(54) **METHOD FOR DIAGNOSING EARLY ONSET OF ALZHEIMER'S DISEASE OR MILD COGNITIVE IMPAIRMENT**
VERFAHREN ZUR DIAGNOSE EINER FRÜHEN FORM VON MORBUS ALZHEIMER ODER LEICHTER KOGNITIVER BEEINTRÄCHTIGUNG
PROCÉDÉ DE DIAGNOSTIC DE L'APPARITION PRÉCOCE DE LA MALADIE D'ALZHEIMER OU D'UN TROUBLE COGNITIF LÉGER

(30) Priority: 18.05.2015 KR 20150068690
(43) Date of publication of application: 28.03.2018
(73) Proprietor: Ant Labs Inc., Seoul 03127 (KR)
(72) Inventor: LEE, Sang Kun, Seoul 06516 (KR); KIM, Man Ho, Seoul 06305 (KR); CHU, Kon, Seoul 05834 (KR); JUNG, Keun Hwa, Seoul 04423 (KR); LEE, Soon Tae, Seoul 04322 (KR); MOON, Jang Sup, Seoul 06705 (KR)
(74) Representative: Schwarz & Partner Patentanwälte OG
(86) International application number: PCT/KR2016/004884
(87) International publication number: WO 2016/186360

(56) References cited:
- EP-A2- 2 617 433
- KR-A- 20120 088 009
- KR-A- 20130 140 528
- KR-A- 20140 044 375
- KR-A- 20150 014 037
- US-A1- 2005 261 218
- US-A1- 2012 094 295
- SOON-TAE LEE ET AL: "miR-206 regulates brain-derived neurotrophic factor in Alzheimer disease model", ANNALS OF NEUROLOGY, vol. 72, no. 2, 1 August 2012 (2012-08-01) , pages 269-277, XP055104571, ISSN: 0364-5134, DOI: 10.1002/ana.23588
- YAMAGISHI M ET AL: "Pathology of olfactory mucosa in patients with Alzheimer's disease", ANNALS OF OTOLOGY, RHINOLOGY AND LARYNGO, ANNALS PUBL., ST. LOUIS, MO, US, vol. 103, no. 6, 1 June 1994 (1994-06-01), pages 421-427, XP009181065, ISSN: 0003-4894, DOI: 10.1177/000348949410300601
- LEE JAE HONG ET AL: "Tau proteins are abnormally expressed in olfactory epithelium of Alzheimer patients and developmentally regulated in human fetal spinal cord", EXPERIMENTAL NEUROLOGY, ELSEVIER, AMSTERDAM, NL, vol. 121, no. 1, 1 January 1993 (1993-01-01), pages 93-105, XP002320958, ISSN: 0014-4886, DOI: 10.1006/EXNR.1993.1074
- STEVEN E. ARNOLD ET AL: "Olfactory epithelium amyloid-&bgr; and paired helical filament-tau pathology in Alzheimer disease", ANNALS OF NEUROLOGY, vol. 67, no. 4, 1 April 2010 (2010-04-01), pages 462-469, XP055034996, ISSN: 0364-5134, DOI: 10.1002/ana.21910
- JANGSUP MOON ET AL: "Early diagnosis of Alzheimer's disease from elevated olfactory mucosal miR-206 level", SCIENTIFIC REPORTS, vol. 6, no. 1, 4 February 2016 (2016-02-04), XP055505738, DOI: 10.1038/srep20364

<110> ANT Labs Inc
<120> METHOD FOR DIAGNOSIS OF EARLY ALZHEIMERS DISEASE OR MILD COGNITIVE IMPAIRMENT
<130> P15001-ANT
<160> 1
<170> KopatentIn 2.0
<210> 1
   <211> 22
   <212> RNA
   <213> Homo sapiens
<400> 1
   uggaauguaa ggaagugugu gg 22

## Description

### TECHNICAL FIELD

The present invention relates to a method for diagnosis of early Alzheimer's disease or mild cognitive impairment.

### BACKGROUND ART

Alzheimer's disease is a brain disease accompanied with aging, and is known to cause apoptosis of neural cells over the whole brain by age-related spots of extracellular beta-amyloid deposition and intracellular hyper phosphorylated tau protein, which cause illness of gradual memory disorder, cognition loss, change of individual personality, and the like. Though three million five hundred thousand or more are suffering from Alzheimer's disease all over the world, it is actual state that drug having effect of improvement on the disease with approval from US FDA has not been developed yet.

Early diagnosis of patient's early stage of Alzheimer's disease is indispensable for curing patient, and is needed in planning clinical test on potential therapeutic agent for Alzheimer's disease.

However, despite studies on pathogenesis through numerous studies up to now and a vast investment in development of therapeutic agent, it is actual state that Alzheimer's disease is still one for which early diagnosis is difficult, and has no reliable bio marker for early diagnosis.

For the reason said above, though a variety of diagnostic method and R&D concerned to the same are undergoing, early diagnosis of Alzheimer's disease is difficult with methods known up to now. For example, despite rapid development of CSF markers and a variety of types of nerve imaging technologies, each of the bio markers has limitations, and above all, has limitation that distinguishing patients having weak cognitive impairment from normal persons in cognitive function is difficult. And, CSF A42 measure and amyloid PET image are markers of amyloid deposition, while amyloid cultivation cannot provide reliable clinic diagnosis result.

That is, brain image diagnosis using MRI (magnetic resonance imaging) or CT (computed tomography), diagnosis measuring amount of tau protein, or beta-amyloid from cerebrospinal fluid (CSF) or blood, APOE-e4 genotype polymorphism analysis as a genetic risk factor, and the like are used for method for diagnosis of Alzheimer's disease, which still have limitations as a method for Alzheimer early diagnosis.

Under these backgrounds, actual state is that a method for early diagnosis of Alzheimer's disease and prognosis prediction thereof is urgently wanted which is based on novel bio marker capable of early diagnosis of Alzheimer's disease and on investigation of method for diagnosis using the same.

EP 2 617 433 A2 discloses a method for detecting a marker for Alzheimer's disease, comprising detecting the expression of miR-424*, miR-18b*, miR-135a*, miR-1228, miR-320, miR-296-5p, miR-557, miR-338-5p, miR-206, miR-92a, miR-1238, miR-513a-5p, miR-423-5p, miR-188-5p, miR-140-3p, miR-575, miR-640, miR-1237, miR-191* or miR-134 in human biological samples, whereby information for diagnosing or predicting a neurodegenerative disease is provided.

### DISCLOSURE

### Technical Problem

The present invention is defined in the appended claims. The present invention is to provide a method for detecting marker for diagnosis of early Alzheimer's disease or mild cognitive impairment.

The present invention is further to provide a method for providing information for diagnosis of early Alzheimer's disease or mild cognitive impairment.

### Technical Solution

To settle the technical problem described above, the present invention includes a method for detecting marker for diagnosis of early Alzheimer's disease or mild cognitive impairment with CDR (Clinic Dementia Rating) 0.5-1.0, through detecting expression of miR-206 existing in olfactory tissue sample for the diagnosis of the early Alzheimer's disease or the mild cognitive impairment with distinguishing cognitive impairment from depression.

And, in the present invention, the detection of expression of the miR-206 is by using primer set, probe or antisense nucleotide specifically binding to the miR-206.

And, in the present invention, the detection of expression of the miR-206 is by reverse transcriptase polymerase reaction, competitive reverse transcriptase polymerase reaction, real-time reverse transcriptase polymerase reaction, RNase protection assay, Northern Blotting or DNA chip.

And, in the present invention, the olfactory tissue sample is olfactory mucosal membrane sample or olfactory epithelium sample.

And, the present invention includes a method for providing information for diagnosis of early Alzheimer's disease or mild cognitive impairment with CDR(Clinic Dementia Rating) 0.5-1.0 including steps of measuring expression level of miR-206 in olfactory tissue sample separated from a patient; and comparing the expression level of the miR-206 to a reference level, for providing information for the diagnosis of the early Alzheimer's disease or the mild cognitive impairment with distinguishing cognitive impairment from depression.

And, in the present invention, it is characterized in further including step of judging diagnosis of Alzheimer's disease or mild cognitive impairment when the expression is increased by comparing the expression level of the miR-206 to the reference level.

And, in the present invention, measurement of the expression level of the miR-206 is by using primer set, probe or antisense nucleotide specifically binding to the miR-206.

And, in the present invention, the detection of the expression of the miR-206 is by reverse transcriptase polymerase reaction, competitive reverse transcriptase polymerase reaction, real-time reverse transcriptase polymerase reaction, RNase protection assay, Northern Blotting or DNA chip.

And, in the present invention, the olfactory tissue sample is olfactory mucosal membrane sample or olfactory epithelium sample.

### Advantageous effects

The method for diagnosis of early Alzheimer's disease or mild cognitive impairment of the present invention identifies expression level of miR-206 of olfactory tissue, by which it shows high diagnostic rate of diagnosis of Alzheimer's disease and mild cognitive impairment, enables diagnosis at low cost, and facilitates high stability of biopsy, causing to show marked effect in diagnosis of Alzheimer's disease or mild cognitive impairment.

Therefore, through therapy and precaution based on the above, curing effect for Alzheimer's disease or mild cognitive impairment can be increased and side effect of curing can be minimized.

### DESCRIPTION OF DRAWINGS

FIG. 1 is a diagram showing miRNA-206 real time PCR result of olfactory epithelium sample.
FIG. 2 is a diagram showing correlation analysis result between olfactory epithelium miRNA-206 level and clinic point measurement result.

### DETAILED DESCRIPTION

Advantages and features of the present invention, and methods for realizing the same may be clarified with reference to embodiments described in detail later. However, the present invention may not be limited to embodiments disclosed hereinafter.

The inventors have been made every effort on discovering a bio marker for early diagnosis of Alzheimer's disease and development of method for diagnosis thereof, and as a result, have identified that expression level of miRNA-206 (miR-206) in olfactory tissue shows a marked effect as a bio marker for diagnosis of early Alzheimer's disease, to complete the present invention.

The present invention provides a method for detecting marker for diagnosis of early Alzheimer's disease or mild cognitive impairment, to provide information needed in diagnosis of Alzheimer's disease or mild cognitive impairment, via method for detecting expression of miR-206 existing in olfactory tissue sample.

In the present invention, diagnosis includes judging susceptibility of an object on specific illness or disease, judging whether an object currently have specific illness or disease or not, judging prognosis (for example, judgement of stage of Alzheimer's disease or judgement of reactivity on curing) of an object getting specific illness or disease, or therametrics (for example, monitoring state of object to provide information on curing effectiveness).

Alzheimer's disease is a brain disease accompanied with aging, and is known to cause apoptosis of neural cells over the whole brain by age-related spots of extracellular beta-amyloid deposition and intracellular hyper phosphorylated tau protein, which cause illness of gradual memory disorder, cognition loss, change of individual personality, and the like.

Mild cognitive impairment means a state in which cognitive function, especially faculty of memory became poor comparing to the same age band, and a state of not in dementia yet since ability to perform ordinary life is preserved. Mild cognitive impairment is pointed out as a high risky group which can shift to Alzheimer's disease, and is a stage in which Alzheimer's disease can be found in an earliest time, and a clinically important stage during which curing effect can be maximized.

According to the present invention, early stage of Alzheimer's disease may be shown by CDR, according to which CDR 0.5 may be defined as very mild dementia stage and/or mild cognitive impairment, and CDR 1, mild dementia.

And, standard object may be classified according to other definition having specific stages of Alzheimer's disease according to known method to ordinary persons in the art.

To provide information needed in diagnosis of early Alzheimer's disease or mild cognitive impairment or in analysis of prognosis of the present invention, method for detecting expression of miR-206 existing in olfactory tissue sample is used.

In the present invention, olfactory tissue sample is a tissue configuring olfactory epithelium, placed at anterior nasal septum. The olfactory tissue sample may be one collected from animal, desirably from human. The olfactory tissue sample is not limited to the above, but desirably is mucosal membrane or epithelium. For example, epithelium cell smeared out by strip off olfactory mucosal membrane with brush may be used, or epithelium tissue collected via operation may be used. These samples may be processed properly according to known method to the field of art for measuring expression level.

The method for detecting expression of miR-206 according to the present invention may use primer set, probe or antisense nucleotide specifically binding to miR-206 maturation sequence, desirably to miR-206 sequence having nucleotide sequence of sequence number 1.

In the present invention, measurement of expression level may include measurement of miRNA-206 expression level. The "measurement of miRNA-206 expression level" in the present invention is, to provide information needed in diagnosis of Alzheimer's disease or mild cognitive impairment or in analysis of prognosis, a process for identifying whether existence of miRNA-206 in sample and extent of expression, which may be known by measuring amount of miRNA-206. For analysis method for the above, reverse transcriptase polymerase reaction, competitive reverse transcriptase polymerase reaction, real-time reverse transcriptase polymerase reaction, RNase protection assay, Northern Blotting or DNA chip, or the like may be raised, but not limited to the same.

For example, expression level of miRNA-206 may be measured via quantitative real-time RT-PCR. And, after measuring CT of each gene (cycle number needed to realize reference value) and calculating ΔCT value (CT of each marker - average CT of reference gene), expression amount of miRNA-206 may be quantified as expression amount of 2^{-ΔCt}.

In the present invention, the method used in the detection method is desirably a quantitative real-time RT-PCR, and commercialized miR-206 detection primer (for example, TM:000510, Applied biosystem Inc. etc.) may be used.

The present invention further provides a method for providing information for diagnosis of Alzheimer's disease or mild cognitive impairment including steps of measuring expression level of miR-206 in olfactory tissue sample separated from a patient; and comparing the expression level of the miR-206 to a reference level.

The method of providing information for diagnosis of Alzheimer's disease or mild cognitive impairment according to the present invention shows remarkable effect in diagnosis of Alzheimer's disease or mild cognitive impairment, and may have marked effect in providing information for diagnosis of especially early Alzheimer's disease including mild cognitive impairment.

The method for providing information for diagnosis of Alzheimer's disease or mild cognitive impairment according to the present invention may include step of measuring expression level of miR-206 in olfactory tissue sample separated from a patient, for which the content about method for detecting expression of above said miR-206 can be applied within the scope of the present invention.

The method for providing information for diagnosis of Alzheimer's disease or mild cognitive impairment according to the present invention may further include step of comparing the expression level of the miR-206 to a reference level.

In the present invention, reference level means the same level as an expression level of gene capable of showing predictability of high diagnosis detected and determined from reference samples by the method written in the present invention. The reference level like above may be induced from statistical analysis of group and/or riskiness prediction data yielded from mathematical algorithm and calculated index. Specific coefficient which becomes reference may be also configured and used using statistical and structural classification algorithm and other method.

In some aspects of embodiments, the term of "reference level" in the present invention means a pre-measured value, and for example, an ordinary person in the art may be set to satisfy requirements in view of specificity, sensitivity, and/or accuracy with reference level being measured in advance. For example, each of sensitivity or specificity may be set with a limit, for example, as 80%, 90% or 95%, and these requirements may be defined in view of prediction value of positive or negative. The reference value may be measured in advance for healthy entity, and may be measured in advance from disease entity to which the patient pertains.

In the present invention, to determine reference level of expression level, a statistical analysis method such as computation of average value or analysis of ROC curve may be used.

The analysis of ROC curve according to an aspect of embodiment of the present invention is for curve showing performance of diagnosis, configured with sensitivity, specificity, and accuracy. In the present invention, sensitivity represents a ratio of the positive among human having Alzheimer's disease, specificity a ratio of the negative among human not having Alzheimer's disease, and accuracy a hit ratio of test result among overall cases. Here, since accuracy of test become high when both of specificity and sensitivity are all high, in ROC curve, x axis becomes 1-specificity, and y axis becomes sensitivity, and AUC (area under curve) representing accuracy means area under the curve.

According to an embodiment of the present invention, reference level is cutoff value showing effect of high prediction and diagnosis on prediction and diagnosis of Alzheimer's disease. For prediction and diagnosis of Alzheimer's disease, expression amount of miR-206 predicts that occurrence of Alzheimer's disease is high for sample in which AUC value in analysis of ROC curve of miR-206 is at middle level (about 0.6 level) or more, more desirably, at reference level with gene expression amount for which AUC value is the highest level being cutoff value.

In the present invention, the term "the same or greater than" or "over" means level exceeding reference level, or overall increase of 1%, 2%, 5%, 10%, 20%, 25%, 30%, 40%, 50%, 60%, 70%, 80%, 85%, 90%, 95%, 96%, 97%, 98%, 99% or more from expression level detected by method written in the present invention comparing to expression level of reference sample. In the present invention, the term "the same or smaller than" or "below" means level under reference level, or overall decrease of 1%, 2%, 5%, 10%, 20%, 25%, 30%, 40%, 50%, 60%, 70%, 80%, 85%, 90%, 95%, 96%, 97%, 98%, 99% or more from expression level detected by method written in the present invention comparing to expression level of reference sample.

The method of providing information for diagnosis of Alzheimer's disease or mild cognitive impairment of the present invention may further include step of comparing expression level of the miR-206 to reference level and, when the expression is increased, judging as diagnosis of Alzheimer's disease or mild cognitive impairment.

The present disclosure also may provide composition for diagnosis of early Alzheimer's disease or mild cognitive impairment including primer set, probe or antisense nucleotide specifically binding to the gene as an agent for measuring expression level of miR-206.

The composition for diagnosis of early Alzheimer's disease or mild cognitive impairment can perform diagnosis and prediction on early Alzheimer's disease or mild cognitive impairment by measuring expression level of miR-206.

The composition for diagnosis may include any one or more of primer set, probe or antisense nucleotide specifically binding to the gene. From nucleotide sequence provided by the present invention, about the primer set, probe or antisense nucleotide, sequence may be designed with ease by an ordinary technical person in the art. According to an embodiment of the present invention, agent for measuring expression level of the miRNA-206 may have, as primer pair, nucleotide sequence of sequence number 2 and sequence number 3.

The present disclosure may also provide kit for diagnosis of early Alzheimer's disease or mild cognitive impairment including composition for diagnosis of early Alzheimer's disease or mild cognitive impairment including primer set, probe or antisense nucleotide specifically binding to the gene as an agent for measuring expression level of miR-206.

The kit can detect by identifying expression level of any one or more of polynucleotide coding the protein. The kit of the present invention may include not only primer of probe for measuring expression level for diagnosis of early Alzheimer's disease or mild cognitive impairment, but also one or more other configuration component composition or device.

Preferably, diagnosis kit for quantitative detection of miR-206 may include oligonucleotide of a kind or more specifically binding to the polynucleotide, and may include primer, reverse transcriptase, Taq polymerase, primer for PCR and dNTP corresponding to these partial sequence, and to measure expression level of polynucleotide, kit using analysis method described in relation to the "measurement of expression level of miRNA-206" can be used. For example, the kit may be selected from RT-PCR kit, competitive RT-PCR kit, real-time RT-PCR kit, real-time RT-PCR kit or DNA chip kit.

### <embodiment 1> patient recruitment and clinic assessment

Experiment was performed after consent from patients using study protocol approved by examination committee of clinical test of Seoul national university hospital. Experiment objects of age of fifty or more was included in this study. Patients having cognitive impairment were recruited from outpatient clinic of the department of neurology of Seoul national university hospital, while participants having general cognitive function ware recruited through advertisement. To assess cognitive function, all the patients received Korean version clinic dementia rating(CDR), Mini-Mental State Examination(MMSE-K) and Alzheimer's Disease Assessment Scale-cognitive subscale(ADAS-cog-K). Beck Depression Inventory-II (BDI-II) was performed for all the patients to judge cognitive impairment by depression. Patients having moderate to severe depression was classified to depression group regardless of score of cognitive function. Patients satisfying criteria for possibility of Alzheimer's disease of national association of neuropathy and communication disorder and stroke and association of Alzheimer's disease and related disease are classified to dementia group, while persons having general cognitive function (CDR 0) to control group. Basic olfactory function was assessed using Korean olfactory test (Korean version of the Sniffing stick test, KVSS).

### <embodiment 2> nasal cavity biopsy of olfactory epithelium

The nasal cavity biopsy was performed at ear-nose-and-throat department of Seoul national university hospital. Cotton gauze humidified by mixture of 2% lidocaine and 0.1% epinephrine was inserted into chapped crack with respect to contraction of blood vessel and local anesthesia. Biopsy was performed by a trained specialist of ear-nose-and-throat department according to endoscope guidance. The tissue sample was collected from anterior nasal septum. Using small curette or small tweezers, two or three 2 mm tissue blocks were collected from nostril. Nasal cavity packing was performed to control bleeding for 10 minutes after biopsy. Experiment objects were clinically observed for 15 to 10 minutes, and all the patients received KVSS before biopsy for assessment with respect to basic olfactory function.

And, in result of checking stability of olfactory epithelium biopsy, most nosebleed was controlled after 30 minutes of biopsy. And, all the patients do not have acute complications, and most patients felt no inconvenience, by which it was verified that stability in collection of tissue sample was secured.

### <embodiment 3> real time PCR of miRNA-206

Total RNA was separated from nosal mucosal membrane tissue using Trizol (Invitrogen, USA), and miRNA was separated using Ambion mirVana isolation kit. RT-PCR was performed using primer set with respect to mirVana qRT-PCR miRNA detection kit and miR-206(**TM:000510**) and U6 (**TM:001973**) snRNA (Applied Biosystems, USA).

All the RT-PCR reaction was conducted in triplicate on an ABI PRISM 7000 sequence detection system (Applied Biosystems, USA), and 40 cycle amplification was performed. Relative expression was calculated using the comparative threshold cycle, and level of miRNA was normalized with respect to U6 level.

### <embodiment 4> result of basic characteristics analysis

Result of analysis with respect to basic characteristics of experiment objects at above embodiment 1 to embodiment 3 was as following table 1:
41 objects were registered in analysis, and objects were categorized based on CDR score and BDI-II score thereof. And, nine patients who were cognitively healthy and not showing depression were assigned to CDR 0 group (control group). Thirteen patients showing weak cognitive impairment were assigned to CDR 0.5, while eleven patients showing slightly stronger cognitive impairment were assigned to CDR 1. Eight patients having moderate to severe depression were classified to depression group. Ages of patients vary over groups, while sex of patients has no big difference over groups. On the other hand, relative miR-206 level means a value divided by average value of CDR 0 group.

Above matters were arranged and shown in table 1.

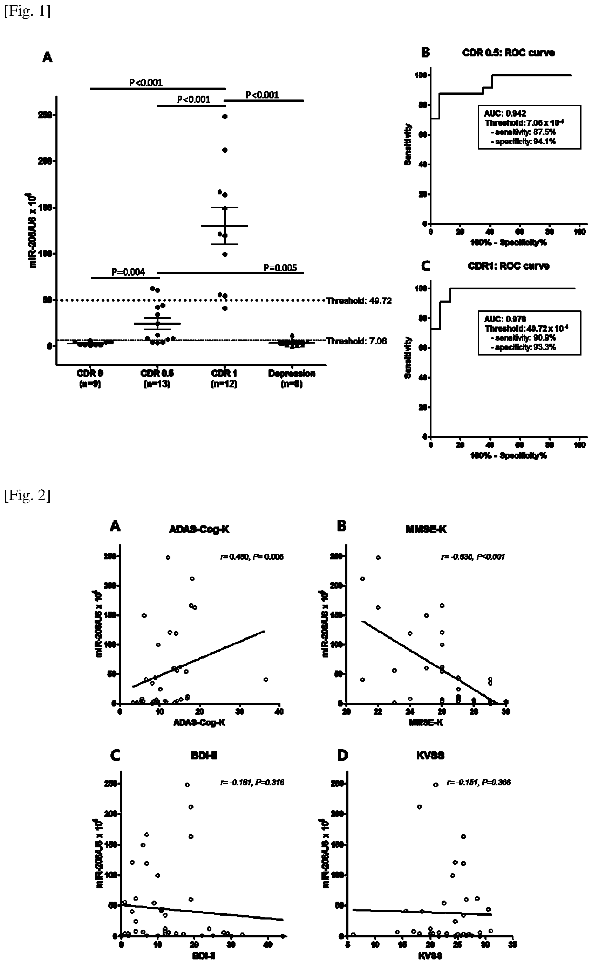

**<table 1>**

| Basic statistical information of patients and clinical result of measurement | | | | | |
|---|---|---|---|---|---|
| | **CDR 0** | **CDR 0.5** | **CDR 1** | **Depression** | **P-value** |
| **Age, average (range)** | 63 (50-74) | 68 (56-79) | 69 (57-80) | 61.5 (56-66) | 0.039 |
| **Sex (Male : Female)** | 4:5 | 4:9 | 6:5 | 2:6 | |
| MMSE-K | 28.32.4 | 26.81.4 | 23.82.1 | 25.14.0 | 0.001 |
| CDR | 0 | 0.5 | 1 | 0.60.4 | |
| ADAS-Cog-K | 8.44.1 | 11.23.9 | 16.07.8 | 14.68.9 | 0.031 |
| BDI-II | 5.25.3 | 11.15.2 | 9.36.6 | 29.46.9 | <0.001 |
| KVSS | 24.23.3 | 23.65.7 | 22.63.1 | 21.97.3 | 0.750 |
| **Relative miR-206 level** | 1 | 7.8 | 41.5 | 1.2 | <0.001 |

As known from the result, it was identified that CDR 0.5 and CDR 1 groups having cognitive disorder comparing to normal control group show remarkably high miR-206 expression level.

### <embodiment 5> identification of increased miR-206 level at olfactory epithelium of patients having early dementia

Result of miRNA RT-PCR of olfactory epithelium sample was shown in FIG. 1.

A of FIG. 1 shows relative miRNA-206 level by each of patients, B of FIG. 1 shows ROC curve by CDR 0.5 group, and C of FIG. 1 shows ROC curve by CDR 1 group.

As shown in A of FIG. 1, at early dementia (early Alzheimer, mild cognitive impairment) group, miR-206 level was largely increased comparing to control group thereof. As shown in A of FIG. 1, increase of 7.8 times was identified by CDR 0.5 group, while increase of 41.5 times was identified by CDR 1 group. That is, tendency of considerable increase according to progress of dementia (Alzheimer, cognitive impairment) was shown. On the other hand, in case of depression group, change in miR-206 level was not shown (1.2 times, not having significance statistically), by which it is identified that there is distinctiveness distinguished from cognitive disorder by depression.

And, Receiver operating characteristic curve was used to determine best cutoff value of relative miR-206 level to identify patients having CDR 0.5 and CDR 1 dementia. The optimal cutoff value was defined as highest sum of sensitivity and specificity, and area under the curve (AUC) was analyzed, by which efficiency of olfactory epithelium miR-206 level with respect to diagnosis of CDR 0.5 or CDR 1 dementia was assessed. The result was shown in B of FIG. 1 and C of FIG. 1.

As shown in B of FIG. 1, AUC value for CDR 0.5 dementia diagnosis was 0.942, and when relative miR-206 expression with respect to U6 exceeds 7.06×10⁻⁴, sensitivity was 87.5 %, while specificity was 94.1 %. Anyone in control group in which cognitive function is normal did not show higher miR-206 level than this level.

And, as shown in C of FIG. 1, AUC value for CDR 1 dementia diagnosis was 0.976, and when relative miR-206 expression with respect to U6 exceeds 49.72×10⁻⁴, sensitivity was 90.9 %, while specificity was 93.3 %. Anyone in control group or depression group did not show miR-206 value exceeding the value.

From the result, it was identified that miR-206 value identified from olfactory tissue has remarkable diagnosis effect on early Alzheimer and mild cognitive impairment.

### <embodiment 6> correlation of olfactory epithelium miR-206 level and clinic score

Since depress is known to give influence to cognitive function, correlation between cognitive function score and relative miR-206 level was assessed for patients not having depression. The result was shown in FIG. 2.

As shown in A and B of FIG. 2, considerable correlation between olfactory epithelium miR-206 level and cognitive function score was identified. That is, it was identified that relative miR-206 level has positive correlation with ADAS-Cog-K score (A of FIG. 2), while has negative correlation with MMSE (B of FIG. 2).

On the other hand, correlation between BDI-II and KVSS score and relative miR-206 level was assessed at all the patients, and the result was shown in C and D of FIG. 2.

As shown in C and D of FIG. 2, relative miR-206 level was not identified as to have correlation with BDI-II and KVSS score.

From the result, it was identified that olfactory epithelium miR-206 level has remarkable effect in diagnosis of early Alzheimer's disease and mild cognitive impairment, with distinguishing cognitive impairment by depression.
## Claims

1. A method for detecting marker for the diagnosis of early Alzheimer's disease or mild cognitive impairment with CDR (Clinic Dementia Rating) 0.5-1.0 comprising:
detecting expression of miR-206 existing in olfactory tissue sample for the diagnosis of the early Alzheimer's disease or the mild cognitive impairment with distinguishing cognitive impairment from depression.

2. The method according to claim 1, wherein
the detection of expression of miR-206 is carried out by using a primer set, probe or antisense nucleotide specifically binding to miR-206.

3. The method according to claim 1, wherein,
the detection of expression of the miR-206 is carried out by reverse transcriptase polymerase reaction, competitive reverse transcriptase polymerase reaction, real-time reverse transcriptase polymerase reaction, RNase protection assay, Northern Blotting or DNA chip.

4. The method according to claim 1, wherein,
the olfactory tissue sample is an olfactory mucosal membrane sample or an olfactory epithelium sample.

5. A method for providing information for diagnosis of early Alzheimer's disease or mild cognitive impairment with CDR(Clinic Dementia Rating) 0.5-1.0 comprising steps of
measuring expression level of miR-206 in olfactory tissue sample separated from a patient; and comparing the expression level of the miR-206 to a reference level for providing information for the diagnosis of the early Alzheimer's disease or the mild cognitive impairment with distinguishing cognitive impairment from depression.

6. The method according to claim 5, further comprising step of
judging diagnosis of early Alzheimer's disease or mild cognitive impairment by distinguishing cognitive disorder from depression, when the expression level is higher that the reference level when comparing the expression level of the miR-206 to the reference level.

7. The method according to claim 5, wherein
the expression level of the miR-206 is measured by using a primer set, probe or antisense nucleotide specifically binding to the miR-206.

8. The method according to claim 5, wherein
the expression level of the miR-206 is measured by reverse transcriptase polymerase reaction, competitive reverse transcriptase polymerase reaction, real-time reverse transcriptase polymerase reaction, RNase protection assay, Northern Blotting or DNA chip.

9. The method according to claim 5, wherein
the olfactory tissue sample is an olfactory mucosal membrane sample or an olfactory epithelium sample.

## Patentansprüche

1. Verfahren zur Detektion eines Markers zur Diagnose von frühem Morbus Alzheimer oder leichter kognitiver Beeinträchtigung mit CDR (Clinical Dementia Rating) von 0,5-1,0, umfassend:
Detektieren der Expression von miR-206, die in olfaktorischer Gewebeprobe vorhanden ist, zur Diagnose von frühem Morbus Alzheimer oder leichter kognitiver Beeinträchtigung mit Unterscheidung von kognitiver Beeinträchtigung von Depression.

2. Verfahren gemäß Anspruch 1, wobei
die Detektion der Expression von miR-206 unter Verwendung eines Primer-Sets, einer Sonde oder eines spezifisch an miR-206 bindenden Antisense-Nukleotids ausgeführt wird.

3. Verfahren gemäß Anspruch 1, wobei
die Detektion der Expression von miR-206 mittels Reverse-Transkriptase-Polymerase-Reaktion, kompetitiver Reverse-Transkriptase-Polymerase-Reaktion, Echtzeit-Reverse-Transkriptase-Polymerase-Reaktion, RNase-Schutz-Assay, Northern Blotting oder DNA-Chip ausgeführt wird.

4. Verfahren gemäß Anspruch 1, wobei
die olfaktorische Gewebeprobe eine olfaktorische Schleimhautmembranprobe oder eine olfaktorische Epithelprobe ist.

5. Verfahren zur Bereitstellung von Information zur Diagnose eines frühen Morbus Alzheimer oder leichter kognitiver Beeinträchtigung mit CDR (Clinical Dementia Rating) von 0,5-1,0, umfassend die folgenden Schritte:
Messen des Expressionsspiegels von miR-206 in einer olfaktorischen Gewebeprobe, die einem Patienten entnommen wurde; und
Vergleichen des Expressionsspiegels der miR-206 mit einem Referenzspiegel zur Bereitstellung von Information zur Diagnose des frühen Morbus Alzheimer oder leichter kognitiver Beeinträchtigung mit Unterscheidung von kognitiver Beeinträchtigung von Depression.

6. Verfahren gemäß Anspruch 5, ferner umfassend die folgenden Schritte:
Beurteilen der Diagnose von frühem Morbus Alzheimer oder leichter kognitiver Beeinträchtigung durch Unterscheidung von kognitiver Störung von Depression, wenn der Expressionsspiegel höher als der Referenzspiegel ist beim Vergleich des Expressionsspiegels der miR-206 mit dem Referenzspiegel.

7. Verfahren gemäß Anspruch 5, wobei
der Expressionsspiegel der miR-206 unter Verwendung eines Primer-Sets, einer Sonde oder eines spezifisch an miR-206 bindenden Antisense-Nukleotids gemessen wird.

8. Verfahren gemäß Anspruch 5, wobei
der Expressionsspiegel der miR-206 mittels Reverse-Transkriptase-Polymerase-Reaktion, kompetitiver Reverse-Transkriptase-Polymerase-Reaktion, Echtzeit-Reverse-Transkriptase-Polymerase-Reaktion, RNase-Schutz-Assay, Northern Blotting oder DNA-Chip gemessen wird.

9. Verfahren gemäß Anspruch 5, wobei
die olfaktorische Gewebeprobe eine olfaktorische Schleimhautmembranprobe oder eine olfaktorische Epithelprobe ist.

## Revendications

1. Procédé de détection d'un marqueur pour le diagnostic de la maladie d'Alzheimer précoce ou d'un trouble cognitif léger avec une CDR (évaluation de démence clinique) de 0,5 à 1,0 comprenant l'étape consistant à :
détecter une expression de miR-206 existant dans un échantillon de tissu olfactif pour le diagnostic de la maladie d'Alzheimer précoce ou du trouble cognitif léger en distinguant le trouble cognitif d'une dépression.

2. Procédé selon la revendication 1, dans lequel
la détection de l'expression de miR-206 est réalisée en utilisant un ensemble d'amorces, une sonde ou un nucléotide antisens se liant spécifiquement à miR-206.

3. Procédé selon la revendication 1, dans lequel,
la détection de l'expression du miR-206 est réalisée par réaction de polymérase-transcriptase inverse, réaction de polymérase-transcriptase inverse compétitive, réaction de polymérase-transcriptase inverse en temps réel, test de résistance à la RNase, transfert de Northern ou puce à ADN.

4. Procédé selon la revendication 1, dans lequel,
l'échantillon de tissu olfactif est un échantillon de membrane de muqueuse olfactive ou un échantillon d'épithélium olfactif.

5. Procédé pour fournir des informations pour le diagnostic de la maladie d'Alzheimer précoce ou d'un trouble cognitif léger avec une CDR (évaluation de démence clinique) de 0,5 à 1,0 comprenant des étapes consistant à
mesurer le niveau d'expression de miR-206 dans un échantillon de tissu olfactif séparé d'un patient ; et
comparer le niveau d'expression du miR-206 à un niveau de référence pour fournir des informations pour le diagnostic de la maladie d'Alzheimer précoce ou du trouble cognitif léger en distinguant le trouble cognitif d'une dépression.

6. Procédé selon la revendication 5, comprenant en outre l'étape d'évaluation du diagnostic de la maladie d'Alzheimer précoce ou d'un trouble cognitif léger en distinguant un trouble cognitif d'une dépression, lorsque le niveau d'expression est supérieur au niveau de référence lors d'une comparaison du niveau d'expression du miR-206 par rapport au niveau de référence.

7. Procédé selon la revendication 5, dans lequel
le niveau d'expression du miR-206 est mesuré en utilisant un ensemble d'amorces, une sonde ou un nucléotide antisens se liant spécifiquement au miR-206.

8. Procédé selon la revendication 5, dans lequel
le niveau d'expression du miR-206 est mesuré par réaction de polymérase-transcriptase inverse, réaction de polymérase-transcriptase inverse compétitive, réaction de polymérase-transcriptase inverse en temps réel, test de résistance à la RNase, transfert de Northern ou puce à ADN.

9. Procédé selon la revendication 5, dans lequel
l'échantillon de tissu olfactif est un échantillon de membrane de muqueuse olfactive ou un échantillon d'épithélium olfactif.
